(19) Europäisches Patentamt
European Patent Office
Office européen des brevets

(11) EP 4 458 948 A1

(12) EUROPEAN PATENT APPLICATION
published in accordance with Art. 153(4) EPC

(43) Date of publication:
**06.11.2024 Bulletin 2024/45**

(21) Application number: **22916003.1**

(22) Date of filing: **26.12.2022**

(51) International Patent Classification (IPC):
***C12M 3/00*** (2006.01) ***C07K 5/09*** (2006.01)
***C07K 5/10*** (2006.01) ***C07K 5/11*** (2006.01)
***C07K 7/06*** (2006.01) ***C07K 17/08*** (2006.01)
***C08F 261/12*** (2006.01) ***C08G 81/02*** (2006.01)
***C08L 101/06*** (2006.01) ***C12M 1/00*** (2006.01)
***C12N 1/00*** (2006.01) ***C12N 5/071*** (2010.01)

(52) Cooperative Patent Classification (CPC):
**C07K 5/0815; C07K 5/10; C07K 5/1019; C07K 7/06; C07K 17/08; C08F 261/12; C08G 81/02; C08L 101/06; C12M 1/00; C12M 3/00; C12N 1/00; C12N 5/06**

(86) International application number:
**PCT/JP2022/047838**

(87) International publication number:
**WO 2023/127780 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2021 JP 2021212493**
**06.01.2022 JP 2022001013**
**06.01.2022 JP 2022001014**
**10.05.2022 JP 2022077686**

(71) Applicant: **SEKISUI CHEMICAL CO., LTD.**
**Osaka-shi**
**Osaka**
**530-8565 (JP)**

(72) Inventors:
- **ARAI, Yuuhei**
**Mishima-gun, Osaka 618-0021 (JP)**
- **NAKAJIMA, Nami**
**Mishima-gun, Osaka 618-0021 (JP)**

(74) Representative: **Ter Meer Steinmeister & Partner**
**Patentanwälte mbB**
**Nymphenburger Straße 4**
**80335 München (DE)**

Remarks:
The complete document including Reference Table(s) and the Sequence Listing(s) can be downloaded from the EPO website

# (54) CELL CULTURE SCAFFOLD MATERIAL

(57) Provided is a scaffold material for cell culture with which the culture stability of cells can be maintained over an extended period of time. A scaffold material for cell culture according to the present invention contains a peptide-conjugated (meth)acrylic copolymer having a (meth)acrylic copolymer moiety and a peptide moiety bonded to the (meth)acrylic copolymer moiety, the following relaxation time ($CS_2$) being 5 milliseconds or less. Relaxation time ($CS_2$): a relaxation time of a CS component, which is a component having the lowest molecular mobility among three components, when the scaffold material for cell culture after being brought into contact with heavy water at 25°C for 18 hours is measured by a CPMG method using pulsed NMR, and the obtained free induction decay curve of spin-spin relaxation of $^1H$ is separated into three components.

[FIG. 1]

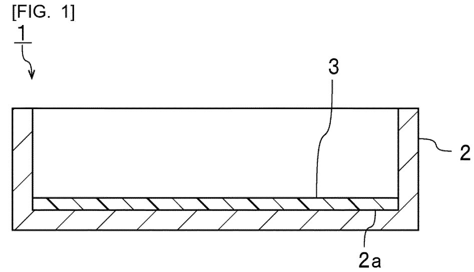


EP 4 458 948 A1

## Description

### TECHNICAL FIELD

The present invention relates to a scaffold material for cell culture.

### BACKGROUND ART

Cells of animals such as humans, mouse, rat, pig, cow, and monkey are used in research and development in academic fields, drug discovery fields, regenerative medicine fields, and the like. As a scaffold material used for culturing animal cells, adhesive proteins such as laminin and vitronectin, and natural polymer materials such as matrigel derived from mouse sarcoma are used.

A scaffold material using a synthetic resin and a scaffold material using a synthetic resin to which a peptide is bonded are also known.

Patent Document 1 below discloses an article for cell culture coated with a composition containing a polymer in which an acrylic polymer and a polypeptide are bonded. In Patent Document 1, a hydrophilic acrylic polymer obtained by polymerizing a hydrophilic acrylic monomer is used as the acrylic polymer.

Patent Document 2 below discloses a coating composition for adhesive cell culture in which a water-insoluble polymer compound is dissolved in a lower alcohol or a mixed solvent of a lower alcohol and water. Patent Document 2 describes, as the water-insoluble polymer compound, a copolymer of a (meth)acrylic acid derivative chemically modified with a peptide and a hydrophilic acrylate compound. As the copolymer, a copolymer having a relatively high content ratio of the hydrophilic acrylate compound is used.

### Related Art Document

### Patent Documents

Patent Document 1: WO 2012/158235 A2
Patent Document 2: JP H5-292957 A

### SUMMARY OF THE INVENTION

### PROBLEMS TO BE SOLVED BY THE INVENTION

As described in Patent Documents 1 and 2, a scaffold material for cell culture using an acrylic copolymer to which a peptide is bonded is known. Cells can be cultured in a liquid medium using a scaffold material, for example, processed or molded into a predetermined shape.

However, in the conventional scaffold materials as described in Patent Documents 1 and 2, since the acrylic copolymer having relatively high hydrophilicity is used, it is easy for the scaffold material to be gradually eluted into the liquid medium during cell culture or for the scaffold material to be detached from a container or the like during cell culture. Therefore, in the conventional scaffold materials as described in Patent Documents 1 and 2, as the number of days of culture of the cells increases, the culture stability of the cells tends to decrease, for example, the proliferation speed of the cells decreases.

An object of the present invention is to provide a scaffold material for cell culture with which the culture stability of cells can be maintained over an extended period of time.

### MEANS FOR SOLVING THE PROBLEMS

According to a broad aspect of the present invention, there is provided a scaffold material for cell culture, containing: a peptide-conjugated (meth)acrylic copolymer having a (meth)acrylic copolymer moiety and a peptide moiety bonded to the (meth)acrylic copolymer moiety, the following relaxation time ($CS_2$) being 5 milliseconds or less.

Relaxation time ($CS_2$) a relaxation time of a CS component, which is a component having the lowest molecular mobility among three components, when the scaffold material for cell culture after being brought into contact with heavy water at 25°C for 18 hours is measured by a CPMG method using pulsed NMR, and the obtained free induction decay curve of spin-spin relaxation of $^1H$ is separated into three components

In a certain aspect of the scaffold material for cell culture according to the present invention, an absolute value

of a difference between the following relaxation time ($SwS_2$) and the following relaxation time ($SdS_2$) is 0.6 milliseconds or less.

Relaxation time ($SwS_2$): a relaxation time of an SwS component, which is a component having lower molecular mobility among two components, when the scaffold material for cell culture after being brought into contact with heavy water at 25°C for 18 hours is measured by a Solid Echo method using pulsed NMR, and the obtained free induction decay curve of spin-spin relaxation of $^1H$ is separated into two components

Relaxation time ($SdS_2$): a relaxation time of an SdS component, which is a component having lower molecular mobility among two components, when the scaffold material for cell culture in a dry state is measured by a Solid Echo method using pulsed NMR, and the obtained free induction decay curve of spin-spin relaxation of $^1H$ is separated into two components

In a certain aspect of the scaffold material for cell culture according to the present invention, the absolute value of the difference between the relaxation time ($SwS_2$) and the relaxation time ($SdS_2$) is 0.01 milliseconds or more and 0.3 milliseconds or less.

In a certain aspect of the scaffold material for cell culture according to the present invention, a water absorption rate is 35 wt% or less.

According to a broad aspect of the present invention, there is provided a scaffold material for cell culture, containing: a peptide-conjugated (meth)acrylic copolymer having a (meth)acrylic copolymer moiety and a peptide moiety bonded to the (meth)acrylic copolymer moiety, a water absorption rate being 35 wt% or less.

In a certain aspect of the scaffold material for cell culture according to the present invention, the (meth)acrylic copolymer moiety has a structural unit derived from a (meth)acrylate compound (A) represented by the following Formula (A1) or the following Formula (A2).

[Chemical 1]

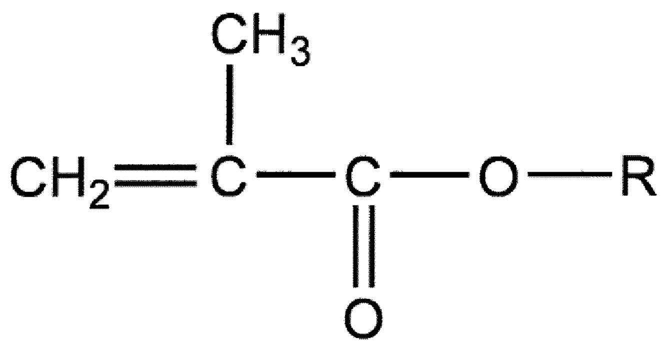

···(A1)

In the Formula (A1), R represents a hydrocarbon group having 2 or more and 18 or less carbon atoms.

[Chemical 2]

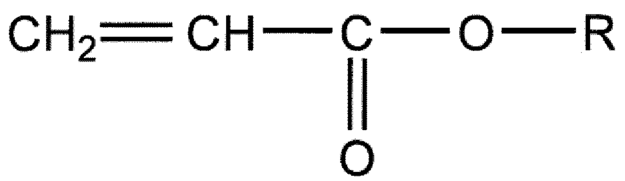

···(A2)

In the Formula (A2), R represents a hydrocarbon group having 2 or more and 18 or less carbon atoms.

In a certain aspect of the scaffold material for cell culture according to the present invention, a content ratio of the structural unit derived from the (meth)acrylate compound (A) is 25 mol% or more and 98 mol% or less in 100 mol% of the total structural units of the (meth)acrylic copolymer moiety.

In a certain aspect of the scaffold material for cell culture according to the present invention, the (meth)acrylic copolymer moiety has a structural unit derived from a (meth)acrylate compound (B) having a functional group capable of reacting with an amino group or a carboxyl group, and in the peptide-conjugated (meth)acrylic copolymer, the peptide moiety is bonded to the functional group capable of reacting with an amino group or a carboxyl group.

In a certain aspect of the scaffold material for cell culture according to the present invention, a number average

molecular weight of the peptide-conjugated (meth)acrylic copolymer is 5000 or more.

In a certain aspect of the scaffold material for cell culture according to the present invention, in the peptide-conjugated (meth)acrylic copolymer, a content ratio of the peptide moiety is 0.5 mol% or more and 25 mol% or less.

In a certain aspect of the scaffold material for cell culture according to the present invention, the peptide moiety has an RGD sequence.

## EFFECT OF THE INVENTION

According to the present invention, it is possible to provide a scaffold material for cell culture with which the culture stability of cells can be maintained over an extended period of time.

## BRIEF DESCRIPTION OF DRAWINGS

[Fig. 1] Fig. 1 is a cross-sectional view schematically illustrating a cell culture container according to an embodiment of the present invention.

[Fig. 2] Fig. 2 is a cross-sectional view schematically illustrating a cell culture microcarrier according to an embodiment of the present invention.

[Fig. 3] Fig. 3 is a view showing a relationship between a relaxation time ($CS_2$) and an absolute value (Z) of a difference between a relaxation time ($SwS_2$) and a relaxation time ($SdS_2$) in scaffold materials for cell culture obtained in Examples and Comparative Examples.

## MODES FOR CARRYING OUT THE INVENTION

Hereinafter, details of the present invention will be described.

A scaffold material for cell culture according to the present invention (hereinafter, may be abbreviated as "scaffold material") contains a peptide-conjugated (meth) acrylic copolymer having a (meth) acrylic copolymer moiety and a peptide moiety bonded to the (meth)acrylic copolymer moiety.

The scaffold material according to the present invention satisfies the following configuration (1) or the following configuration (2).

Configuration (1): The following relaxation time ($CS_2$) is 5 milliseconds or less.

Relaxation time ($CS_2$): a relaxation time of a CS component, which is a component having the lowest molecular mobility among three components, when the scaffold material for cell culture after being brought into contact with heavy water at 25°C for 18 hours is measured by a CPMG method using pulsed NMR, and the obtained free induction decay curve of spin-spin relaxation of $^1H$ is separated into three components.

Configuration (2): A water absorption rate is 35 wt% or less.

Since the scaffold material according to the present invention is provided with the above configuration, the culture stability of cells can be maintained over an extended period of time. In the scaffold material according to the present invention, since the hydrophobicity is adjusted to be relatively large, the scaffold material is less likely to be eluted into a liquid medium during cell culture, and the scaffold material is less likely to be detached from a container or the like during cell culture. Therefore, in the scaffold material according to the present invention, even when cells are cultured for a long period of time, the proliferation speed of the cells is less likely to decrease.

The scaffold material may satisfy the configuration (1), the configuration (2), or the configuration (1) and the configuration (2). From the viewpoint of more effectively exhibiting the effects of the present invention, the scaffold material preferably satisfies the configuration (1) and the configuration (2).

In the scaffold material according to the present invention, solubility in an alcohol solvent such as ethanol can be improved. Therefore, for example, by applying a coating solution obtained by dissolving the scaffold material in ethanol to a surface of a container or the like and then volatilizing the ethanol, a scaffold material layer having a predetermined shape can be formed on the surface of the container or the like. When the solubility in an alcohol solvent is favorable, the concentration of the scaffold material in the coating solution can be increased, so that a scaffold material layer having a large thickness can be formed.

In the scaffold material according to the present invention, since it is not necessary to use a natural polymer material such as an extracellular matrix (ECM) as a material, it is inexpensive, has little variation between lots, and is excellent in safety.

(Pulsed NMR)

<CPMG method>

The CPMG method is a measurement method in which a structural moiety having relatively low crystallinity is a measurement target. The smaller the relaxation time ($CS_2$), the lower the molecular mobility of the CS component after the scaffold material absorbs water, which means that the scaffold material is microscopically less likely to elute into the liquid medium. Therefore, as the relaxation time ($CS_2$) is smaller, the effects of the present invention are more effectively exhibited.

The measurement by the CPMG method using pulsed NMR can be performed as follows.

The coating solution in which the scaffold material is dissolved is cast on a surface of a release-treated substrate, and then dried in an oven at 35°C for 24 hours to obtain a film (scaffold material layer) having a thickness of 0.5 mm and composed of the scaffold material. The coating solution for forming the film is, for example, a solution in which the scaffold material is dissolved in an alcohol solvent such as ethanol. When the scaffold material to be measured is a solid such as a resin film, the scaffold material is dissolved in an alcohol solvent such as ethanol to obtain a coating solution for forming the film. When the concentration of the scaffold material in the coating solution is low, a solution obtained by appropriately volatilizing and concentrating the solvent is used as the coating solution for forming the film.

The obtained film is cut into a square of 1 mm in length and 1 mm in width, 200 mg of the obtained cut piece is placed in an inner glass tube (manufactured by BRUKER) for a tube having a diameter of 10 mm, and 1 mL of heavy water is added. Heavy water and the cut piece (scaffold material) are brought into contact with each other at 25°C for 18 hours to prepare a measurement sample (scaffold material after being brought into contact with heavy water at 25°C for 18 hours).

Next, the measurement sample is installed in a pulsed NMR apparatus ("the minispec mq20" manufactured by BRUKER), held at 25°C for 5 minutes, and then measured by a CPMG method under the following conditions to obtain a free induction decay curve of spin-spin relaxation of $^1H$.

<Measurement conditions of CPMG method>

Scans: 64
Recycle Delay: 0.4 msec
90-180 Pulse Separation (tau): 0.055
Total Number: 5000

The above measurement conditions are an example. Scans is set so that the intensity of the free induction decay curve of spin-spin relaxation of the normalized $^1H$ is 0.02 msec or less, and Recycle Delay is set so as to be 5 times a longitudinal relaxation time T1. 90-180 Pulse Separation (tau) and Total Number are set to values at which the free induction decay curve of spin-spin relaxation of $^1H$ is completely attenuated.

The obtained free induction decay curve of spin-spin relaxation of $^1H$ is separated into three components. More specifically, the obtained free induction decay curve of spin-spin relaxation of $^1H$ is subjected to waveform separation into three curves respectively derived from three components of a CS component, a CM component, and a CL component in ascending order of relaxation time. The CS component is a component having the lowest molecular mobility among the three components. The CL component is a component having the highest molecular mobility among the three components. The CM component is a component having molecular mobility (that is, moderate molecular mobility) between the CS component and the CL component, and thus having a relaxation time between the CS component and the CL component. Waveform separation is performed by fitting using an exponential type. The ratio of each component (CS component, CM component, and CL component) is determined from the obtained curve derived from the three components.

The fitting is performed according to the product manual using analysis software "TD-NMRA (Version 4.3 Rev 0.8)" manufactured by BRUKER. For fitting, the following Formula (P) is used.
[Mathematical 1]

$$Y = A1 \times exp\left(-\frac{1}{w1} \times \left(\frac{t}{T_{2A}}\right)^{w1}\right) + B1 \times \exp\left(-\frac{1}{w2} \times \left(\frac{t}{T_{2B}}\right)^{w2}\right) + C1 \times \exp\left(-\frac{1}{w3} \times \left(\frac{t}{T_{2C}}\right)^{w3}\right) \quad \cdots (P)$$

In the above Formula (P), Y is a relaxation strength. In the above Formula (P), w1 to w3 are Weibull coefficients, and w1, w2, and w3 are each 1. In the above Formula (P), A1 is the component ratio (%) of the CS component, B1 is the component ratio (%) of the CM component, C1 is the component ratio (%) of the CL component, $T_{2A}$ is the relaxation time (milliseconds) of the CS component, $T_{2B}$ is the relaxation time (milliseconds) of the CM component, and $T_{2C}$ is the relaxation time (milliseconds) of the CL component. In the above Formula (P), t is time. In the above Formula (P), the sum of A1, B1, and C1 is 100 (%).

The relaxation time ($CS_2$) is preferably 0.1 milliseconds or more, more preferably 0.5 milliseconds or more, still more preferably 1 millisecond or more, further preferably 1.2 milliseconds or more, and particularly preferably 1.5 milliseconds or more, and is preferably 5 milliseconds or less, more preferably 4.5 milliseconds or less, still more preferably 2 milliseconds or less, and particularly preferably 1.9 milliseconds or less. When the relaxation time ($CS_2$) is the lower limit or more, the solubility in an alcohol solvent such as ethanol can be further improved, and thus coatability and processability can be further improved. When the relaxation time ($CS_2$) is the upper limit or less and the upper limit or less, the effect of the present invention can be more effectively exhibited.

Examples of a method for reducing the relaxation time ($CS_2$) include the following methods: (1) a method of increasing the content ratio of a structural unit derived from a hydrophobic (meth)acrylate compound in the peptide-conjugated (meth)acrylic copolymer; (2) a method of using a (meth)acrylate compound having a hydrophobic group with high hydrophobicity as a material of the peptide-conjugated (meth)acrylic copolymer; and (3) a method of increasing the molecular weight of the peptide-conjugated (meth)acrylic copolymer.

<Solid Echo method>

In the scaffold material, it is preferable to perform measurement by a Solid Echo method using pulsed NMR. In this case, from the viewpoint of more effectively exhibiting the effects of the present invention, an absolute value in difference between the following relaxation time ($SwS_2$) and the following relaxation time ($SdS_2$) is preferably 0.6 milliseconds or less. In the present specification, the absolute value in difference between the relaxation time ($SwS_2$) and the relaxation time ($SdS_2$) may be referred to as an absolute value (Z) of the difference.

Relaxation time ($SwS_2$): a relaxation time of an SwS component, which is a component having lower molecular mobility among two components, when the scaffold material for cell culture after being brought into contact with heavy water at 25°C for 18 hours is measured by a Solid Echo method using pulsed NMR, and the obtained free induction decay curve of spin-spin relaxation of $^1H$ is separated into two components.

Relaxation time ($SdS_2$): a relaxation time of an SdS component, which is a component having lower molecular mobility among two components, when the scaffold material for cell culture in a dry state is measured by a Solid Echo method using pulsed NMR, and the obtained free induction decay curve of spin-spin relaxation of $^1H$ is separated into two components.

The Solid Echo method is a measurement method in which a structural moiety having relatively high crystallinity is a measurement target. The smaller the absolute value (Z) of the difference, the less macroscopically the scaffold material swells in the liquid medium. Therefore, as the absolute value (Z) of the difference is smaller, the effects of the present invention are more effectively exhibited.

The relaxation time ($SwS_2$) can be measured as follows.

A measurement sample (scaffold material after being brought into contact with heavy water at 25°C for 18 hours) is prepared by the same method as in the measurement by the CPMG method using pulsed NMR.

Next, the measurement sample is installed in a pulsed NMR apparatus ("the minispec mq20" manufactured by BRUKER), held at 25°C for 5 minutes, and then measured by a Solid Echo method under the following conditions to obtain a free induction decay curve of spin-spin relaxation of $^1H$.

<Measurement conditions of Solid Echo method>

Scans: 128
Recycle Delay: 0.7 sec
Acquisition scale: 1 msec

The above measurement conditions are an example. Scans is set so that the intensity of the free induction decay curve of spin-spin relaxation of the normalized $^1H$ is 0.02 msec or less, and Recycle Delay is set so as to be 5 times a longitudinal relaxation time T1.

The obtained free induction decay curve of spin-spin relaxation of $^1H$ is separated into two components. More specifically, the obtained free induction decay curve of spin-spin relaxation of $^1H$ is subjected to waveform separation

into two curves respectively derived from two components of an SwS component and an SwL component in ascending order of relaxation time. The SwS component is a component having lower molecular mobility among the two components. The SwL component is a component having higher molecular mobility among the two components. Waveform separation is performed by fitting using a Gaussian type and an exponential type. The ratio of each component (SwS component and SwL component) is determined from the obtained curve derived from the two components.

The fitting is performed according to the product manual using analysis software "TD-NMRA (Version 4.3 Rev 0.8)" manufactured by BRUKER. For fitting, the following Formula (Q) is used. For the analysis, fitting is performed using points up to 0.5 msec of the free induction decay curve of spin-spin relaxation of $^1$H.

[Mathematical 2]

$$\mathrm{Y} = A1 \times \exp\left(-\frac{1}{w1} \times \left(\frac{t}{T_{2A}}\right)^{w1}\right) + B1 \times \exp\left(-\frac{1}{w2} \times \left(\frac{t}{T_{2B}}\right)^{w2}\right) \quad \cdot \cdot \cdot \text{(Q)}$$

In the above Formula (Q), Y is a relaxation strength. In the above Formula (Q), w1 and w2 are Weibull coefficients, w1 is 2, and w2 is 1. In the above Formula (Q), A1 is the component ratio (%) of the SwS component, B1 is the component ratio (%) of the SwL component, $T_{2A}$ is the relaxation time (milliseconds) of the SwS component, and $T_{2B}$ is the relaxation time (milliseconds) of the SwL component. In the above Formula (Q), t is time. In the above Formula (Q), the sum of A1 and B1 is 100 (%).

The relaxation time ($SdS_2$) can be measured in the same manner as in the measurement of the relaxation time ($SwS_2$), except that an operation of adding 1 mL of heavy water to an inner glass tube (manufactured by BRUKER) for a tube having a diameter of 10 mm and bringing the heavy water and the cut piece into contact with each other for 18 hours is omitted. That is, the relaxation time ($SdS_2$) can be measured in the same manner as in the measurement of the relaxation time ($SwS_2$) , except that the cut piece (scaffold material) in a dry state is used as a measurement sample. The SdS component and the SdL component at the relaxation time ($SdS_2$) correspond to the SwS component and the SwL component at the relaxation time ($SwS_2$), respectively.

The absolute value in difference (absolute value (Z) of the difference) between the relaxation time ($SwS_2$) and the relaxation time ($SdS_2$) is preferably 0.01 milliseconds or more, more preferably 0.02 milliseconds or more, still more preferably 0.03 milliseconds or more, and particularly preferably 0.04 milliseconds or more, and is preferably 0.6 milliseconds or less, more preferably 0.3 milliseconds or less, still more preferably 0.2 milliseconds or less, and particularly preferably 0.1 milliseconds or less. When the absolute value (Z) of the difference is the lower limit or more, the solubility in an alcohol solvent such as ethanol can be further improved, and thus coatability and processability can be further improved. When the absolute value (Z) of the difference is the lower limit or more and the upper limit or less, the effect of the present invention can be more effectively exhibited.

Examples of a method for reducing the absolute value (Z) of the difference include the following methods. (1) a method of increasing the content ratio of a structural unit derived from a hydrophobic (meth)acrylate compound in the peptide-conjugated (meth)acrylic copolymer; (2) a method of using a (meth)acrylate compound having a hydrophobic group with high hydrophobicity as a material of the peptide-conjugated (meth)acrylic copolymer; and (3) a method of increasing the molecular weight of the peptide-conjugated (meth)acrylic copolymer.

The relaxation time ($SwS_2$) may be larger or smaller than the relaxation time ($SdS_2$), but usually, the relaxation time ($SwS_2$) is larger than the relaxation time ($SdS_2$).

The relaxation time ($SwS_2$) is preferably 0.01 milliseconds or more, more preferably 0.03 milliseconds or more, still more preferably 0.05 milliseconds or more, and particularly preferably 0.055 milliseconds or more, is preferably 0.6 milliseconds or less, more preferably 0.3 milliseconds or less, still more preferably 0.1 milliseconds or less, and particularly preferably 0.08 milliseconds or less. When the relaxation time ($SwS_2$) is the lower limit or more and the upper limit or less, the effect of the present invention can be more effectively exhibited.

The relaxation time ($SdS_2$) is preferably 0.001 milliseconds or more and more preferably 0.005 milliseconds or more, and is preferably 0.05 milliseconds or less and more preferably 0.03 milliseconds or less. When the relaxation time ($SdS_2$) is the lower limit or more and the upper limit or less, the effect of the present invention can be more effectively exhibited.

(Water absorption rate)

The water absorption rate of the scaffold material is preferably 1 wt% or more, more preferably 5 wt% or more, still more preferably 10 wt% or more, and particularly preferably 20 wt% or more, and is preferably 35 wt% or less, more preferably 30 wt% or less, still more preferably 27 wt% or less, and particularly preferably 25 wt% or less. When the water absorption rate is the lower limit or more, the solubility in an alcohol solvent such as ethanol can be further improved, and thus coatability and processability can be further improved. When the water absorption rate is the upper limit or

less, the effect of the present invention can be more effectively exhibited.

The water absorption rate of the scaffold material can be measured as follows.

The coating solution in which the scaffold material is dissolved is cast on a surface of a release-treated substrate, and then dried in an oven at 35°C for 24 hours to obtain a film (scaffold material layer) having a thickness of 0.5 mm and composed of the scaffold material. The coating solution for forming the film is, for example, a solution in which the scaffold material is dissolved in an alcohol solvent such as ethanol. When the scaffold material to be measured is a solid such as a resin film, the scaffold material is dissolved in an alcohol solvent such as ethanol to obtain a coating solution for forming the film. When the concentration of the scaffold material in the coating solution is low, a solution obtained by appropriately volatilizing and concentrating the solvent is used as the coating solution for forming the film.

The obtained film is cut into a square of 1 mm in length and 1 mm in width, and the obtained cut piece is cooled with liquid nitrogen for 10 minutes. Next, the cooled cut piece is pulverized at 50 Hz for 3 minutes using a freeze pulverizer ("JFC-2000" manufactured by Japan Analytical Industry Co., Ltd.). Next, an operation of cooling with liquid nitrogen for 5 minutes and pulverizing at 50 Hz for 3 minutes using the freeze pulverizer (operation from cooling for 5 minutes to pulverization for 3 minutes) is repeated three times. Next, the pulverized sample of the scaffold material thus obtained is passed through a stainless steel sieve (Product No.: 300×100/300 μm, manufactured by Sanpoh) having an opening of 300 μm. The pulverized sample passed through the sieve is dried in an oven at 35°C for 24 hours to obtain a pulverized sample in a dry state (scaffold material in a dry state). Next, 300 mg of the pulverized sample in a dry state is placed in a weighed glass vial tube (Product No.: 211221 manufactured by Maruemu Corporation), and left to stand still for 24 hours in a constant temperature and humidity oven ("SH-641" manufactured by ESPEC CORP.) at 37°C and a humidity of 90% without a lid. The weight of the pulverized sample after leaving to stand still for 24 hours (scaffold material after water absorption) is measured. The water absorption rate of the scaffold material is calculated by the following formula.

$$\text{Water absorption rate (wt\%)} = (W_2 - W_1)/W_1 \times 100$$

$W_1$: Weight (mg) of pulverized sample in dry state (scaffold material in dry state)
$W_2$: Weight (mg) of pulverized sample after leaving to stand still for 24 hours in constant temperature and humidity oven at 37°C and a humidity of 90% (scaffold material after water absorption)

Examples of a method for reducing the water absorption rate of the scaffold material include the following methods. (1) a method of increasing the content ratio of a structural unit derived from a hydrophobic (meth)acrylate compound in the peptide-conjugated (meth)acrylic copolymer; (2) a method of using a (meth)acrylate compound having a hydrophobic group with high hydrophobicity as a material of the peptide-conjugated (meth)acrylic copolymer; and (3) a method of increasing the molecular weight of the peptide-conjugated (meth)acrylic copolymer.

Hereinafter, details of the scaffold material will be further described. In the present specification, "(meth)acrylic" means one or both of "acrylic" and "methacrylic", and "(meth)acrylate" means one or both of "acrylate" and "methacrylate".

(Peptide-conjugated (meth)acrylic copolymer)

The scaffold material contains a peptide-conjugated (meth)acrylic copolymer. The peptide-conjugated (meth)acrylic copolymer is a (meth)acrylic copolymer to which a peptide is bonded. The peptide-conjugated (meth)acrylic copolymer has a (meth)acrylic copolymer moiety and a peptide moiety bonded to the (meth)acrylic copolymer moiety. Only one kind of the peptide-conjugated (meth)acrylic copolymer may be used, or two or more kinds thereof may be used in combination.

<(Meth)acrylic copolymer moiety>

The (meth)acrylic copolymer moiety preferably has a structural unit derived from a (meth)acrylate compound (A) represented by the following Formula (A1) or the following Formula (A2). Thereby, the hydrophobicity of the peptide-conjugated (meth)acrylic copolymer can be further increased. The relaxation time ($CS_2$), the absolute value (Z) of the difference, and the water absorption rate can be reduced. The (meth)acrylate compound (A) may contain a (meth)acrylate compound represented by the following Formula (A1), may contain a (meth)acrylate compound represented by the following Formula (A2), or may contain both a (meth)acrylate compound represented by the following Formula (A1) and a (meth)acrylate compound represented by the following Formula (A2). When the (meth)acrylate compound (A) contains both a (meth)acrylate compound represented by the following Formula (A1) and a (meth)acrylate compound represented by the following Formula (A2), R in the following Formula (A1) and R in the following Formula (A2) may be the same or different. Only one kind of the (meth) acrylate compound (A) may be used, or two or more kinds thereof may be used in combination. Only one kind of each of the (meth)acrylate compound represented by the following Formula (A1) and

the (meth)acrylate compound represented by the following Formula (A2) may be used, or two or more kinds thereof may be used in combination.

[Chemical 3]

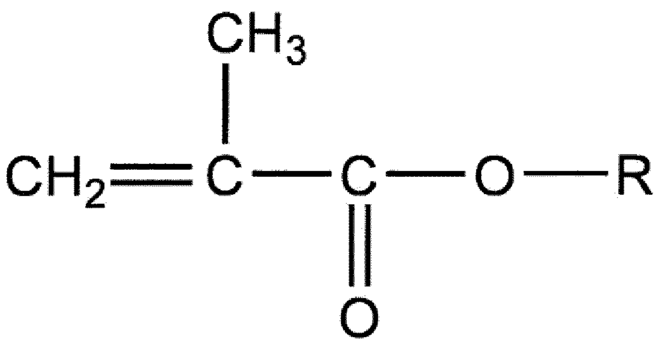

···(A1)

In the above Formula (A1), R represents a hydrocarbon group having 2 or more and 18 or less carbon atoms.

[Chemical 4]

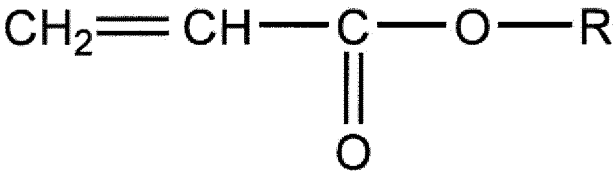

···(A2)

In the above Formula (A2), R represents a hydrocarbon group having 2 or more and 18 or less carbon atoms.

Each of R in the above Formula (A1) and R in the above Formula (A2) may be an aliphatic hydrocarbon group or an aromatic hydrocarbon group. From the viewpoint of further enhancing the solubility of the peptide-conjugated (meth)acrylic copolymer in an alcohol solvent, each of R in the above Formula (A1) and R in the above Formula (A2) is preferably an aliphatic hydrocarbon group. The aliphatic hydrocarbon group may be linear, may have a branched structure, may have a double bond, or may not have a double bond. Each of R in the above Formula (A1) and R in the above Formula (A2) may be an alkyl group or an alkylene group.

The number of carbon atoms of R in the above Formula (A1) and the number of carbon atoms of R in the above Formula (A2) are each preferably 4 or more, more preferably 6 or more, still more preferably 8 or more, and particularly preferably 10 or more, and is preferably 16 or less, more preferably 14 or less, and most preferably 12. When the number of carbon atoms is the lower limit or more, the hydrophobicity of the peptide-conjugated (meth)acrylic copolymer can be further increased. The relaxation time ($CS_2$), the absolute value (Z) of the difference, and the water absorption rate can be reduced. When the number of carbon atoms is the upper limit or less, the solubility in an alcohol solvent such as ethanol can be further improved, and thus coatability and processability can be further improved. In particular, when the number of carbon atoms is 12, the effects of the present invention can be further more effectively exhibited, and coatability and processability can be further enhanced.

The content ratio of the structural unit derived from the (meth) acrylate compound (A) in 100 mol% of the total structural units of the (meth)acrylic copolymer moiety is preferably 10 mol% or more, more preferably 20 mol% or more, more preferably 25 mol% or more, still more preferably 30 mol% or more, further preferably 40 mol% or more, and particularly preferably 50 mol% or more, and is preferably 98 mol% or less, more preferably 95 mol% or less, still more preferably 90 mol% or less, and particularly preferably 80 mol% or less. When the content ratio is the lower limit or more, the hydrophobicity of the peptide-conjugated (meth)acrylic copolymer can be further increased. The relaxation time ($CS_2$), the absolute value (Z) of the difference, and the water absorption rate can be reduced. When the content ratio is the upper limit or less, the solubility in an alcohol solvent such as ethanol can be further improved, and thus coatability and processability can be further improved.

The (meth)acrylic copolymer moiety preferably has a structural unit derived from a (meth)acrylate compound

(B) having a functional group capable of reacting with an amino group or a carboxyl group. The (meth)acrylate compound (B) has a functional group capable of reacting with an amino group or a functional group capable of reacting with a carboxyl group. The (meth)acrylate compound (B) may have a functional group capable of reacting with an amino group, may have a functional group capable of reacting with a carboxyl group, and may have both a functional group capable of reacting with an amino group and a functional group capable of reacting with a carboxyl group. Only one kind of the (meth)acrylate compound (B) may be used, or two or more kinds thereof may be used in combination.

Examples of the functional group capable of reacting with an amino group or a carboxyl group include a carboxyl group, a thiol group, an amino group, and a cyano group.

From the viewpoint of effectively exhibiting the effects of the present invention, in the peptide-conjugated (meth)acrylic copolymer, the peptide moiety is preferably bonded to the functional group capable of reacting with an amino group or a carboxyl group. More specifically, the carboxyl group or amino group of the amino acid constituting the peptide moiety is preferably bonded to the functional group capable of reacting with an amino group or a carboxyl group.

The functional group capable of reacting with an amino group or a carboxyl group is preferably a carboxyl group or an amino group. The (meth)acrylate compound (B) preferably has a carboxyl group or an amino group.

Examples of the (meth)acrylate compound (B) include (meth)acrylic acid, 3-butenoic acid, 4-pentenoic acid, 5-hexenoic acid, 6-heptenoic acid, 7-octenoic acid, benzene acrylic acid, (meth)acryloyloxyethylsuccinic acid, (meth)acryloyloxyethylphthalic acid, (meth)acryloyloxypropylsuccinic acid, (meth)acryloyloxypropylphthalic acid, (meth)acryloyloxyethylhexahydrosuccinic acid, (meth)acryloyloxyethylhexahydrophthalic acid, (meth)acryloyloxypropylhexahydrosuccinic acid, and (meth)acryloyloxypropylhexahydrophthalic acid.

The (meth)acrylate compound (B) is preferably (meth)acrylic acid, (meth)acryloyloxyethylsuccinic acid, (meth)acryloyloxypropylsuccinic acid, (meth)acryloyloxyethylhexahydrosuccinic acid, (meth)acryloyloxypropylhexahydrosuccinic acid, or butenoic acid, and more preferably (meth) acrylic acid. In this case, the effects of the present invention can be more effectively exhibited.

The content ratio of the structural unit derived from the (meth) acrylate compound (B) in 100 mol% of the total structural units of the (meth)acrylic copolymer moiety is preferably 2 mol% or more, more preferably 5 mol% or more, and still more preferably 10 mol% or more, and is preferably 75 mol% or less, more preferably 70 mol% or less, and still more preferably 60 mol% or less. When the content ratio is the lower limit or more, the solubility in an alcohol solvent such as ethanol is easily enhanced. When the content ratio is the upper limit or less, the culture stability of cells is easily maintained over an extended period of time.

The total content ratio of the structural unit derived from the (meth)acrylate compound (A) and the structural unit derived from the (meth)acrylate compound (B) in 100 mol% of the total structural units of the (meth)acrylic copolymer moiety is preferably 50 mol% or more, more preferably 65 mol% or more, still more preferably 80 mol% or more, still further preferably 90 mol% or more, particularly preferably 95 mol% or more, and most preferably 100 mol%. When the total content ratio is the lower limit or more, the effects of the present invention can be more effectively exhibited. The total content ratio may be 100 mol% or less or 90 mol% or less.

The (meth)acrylic copolymer moiety may include a structural unit derived from a (meth)acrylate compound different from both the (meth)acrylate compound (A) and the (meth)acrylate compound (B) as long as it is not contrary to the object of the present invention. The (meth)acrylic copolymer moiety may include a structural unit derived from a vinyl compound copolymerizable with the (meth)acrylate compound as long as it is not contrary to the object of the present invention.

The content ratio of the structural unit derived from the (meth)acrylate compound (A) and the content ratio of the structural unit derived from the (meth)acrylate compound (B) in the (meth)acrylic copolymer moiety can be measured by, for example, nuclear magnetic resonance (NMR).

<Peptide moiety>

The peptide moiety is a structural moiety derived from a peptide. The peptide moiety has an amino acid sequence. The peptide constituting the peptide moiety may be an oligopeptide or a polypeptide. Only one kind of the peptide may be used, or two or more kinds thereof may be used in combination.

The number of amino acid residues in the peptide moiety is preferably 3 or more, more preferably 4 or more, and still more preferably 5 or more, and is preferably 10 or less, more preferably 8 or less, and still more preferably 6 or less. When the number of amino acid residues is the lower limit or more and the upper limit or less, the adhesion to cells after seeding can be further enhanced, and the proliferation rate of cells can be further enhanced. However, the number of amino acid residues in the peptide moiety may be more than 10 or more than 15.

The peptide moiety preferably has a cell adhesive amino acid sequence. The cell adhesive amino acid sequence refers to an amino acid sequence whose cell adhesion activity has been confirmed by a phage display method, a sepharose bead method, or a plate coating method. As the phage display method, for example, the method described in "The Journal of Cell Biology, Volume 130, Number 5, September 1995 1189-1196" can be used. As the sepharose

bead method, for example, the method described in "PROTEIN, NUCLEIC ACID, AND ENZYME, Vol. 45, No. 15 (2000) 2477" can be used. As the plate coating method, for example, the method described in "PROTEIN, NUCLEIC ACID, AND ENZYME, Vol. 45, No. 15 (2000) 2477" can be used.

Examples of the cell adhesive amino acid sequence include an RGD sequence (Arg-Gly-Asp), a YIGSR sequence (Tyr-Ile-Gly-Ser-Arg), a PDSGR sequence (Pro-Asp-Ser-Gly-Arg), an HAV sequence (His-Ala-Val), an ADT sequence (Ala-Asp-Thr), a QAV sequence (Gln-Ala-Val), an LDV sequence (Leu-Asp-Val), an IDS sequence (Ile-Asp-Ser), an REDV sequence (Arg-Glu-Asp-Val), an IDAPS sequence (Ile-Asp-Ala-Pro-Ser), a KQAGDV sequence (Lys-Gln-Ala-Gly-Asp-Val), and a TDE sequence (Thr-Asp-Glu). Examples of the cell adhesive amino acid sequence include the sequences described in "Pathophysiology, Vol. 9, No. 7, p. 527 to 535, 1990" and "Osaka. Medical Center and Research Institute for Maternal and Child Health magazine, Vol. 8, No. 1, p. 58 to 66, 1992". The peptide moiety may have only one type of the cell adhesive amino acid sequence or two or more types thereof.

The peptide moiety preferably has at least one of the above-described cell adhesive amino acid sequences, more preferably has at least an RGD sequence, a YIGSR sequence, or a PDSGR sequence, still more preferably has an RGD sequence, and particularly preferably has at least an RGD sequence represented by the following Formula (1). In this case, the adhesion to cells after seeding can be further enhanced, and the proliferation rate of cells can be further enhanced.

Arg-Gly-Asp-X    Formula (1)

In the above Formula (1), X represents Gly, Ala, Val, Ser, Thr, Phe, Met, Pro, or Asn.

The peptide moiety may be linear or may have a cyclic peptide skeleton. The cyclic peptide skeleton is a cyclic skeleton composed of a plurality of amino acids. From the viewpoint of further effectively exhibiting the effects of the present invention, the cyclic peptide skeleton is preferably composed of four or more amino acids, more preferably composed of five or more amino acids, and preferably composed of ten or less amino acids.

In the peptide-conjugated (meth)acrylic copolymer, the content ratio of the peptide moiety is preferably 0.5 mol% or more, more preferably 1 mol% or more, and still more preferably 5 mol% or more, and is preferably 25 mol% or less, more preferably 20 mol% or less, and still more preferably 15 mol% or less. When the content ratio of the peptide moiety is the lower limit or more, the adhesion to cells after seeding can be further enhanced, and the proliferation rate of cells can be further enhanced. When the content ratio of the peptide moiety is the upper limit or less, the culture stability of cells can be further enhanced, and the production cost can be suppressed. The content ratio (mol%) of the peptide moiety is the amount of substance of the peptide moiety with respect to the total of the amount of substance of structural units constituting the peptide-conjugated (meth)acrylic copolymer.

The content ratio of the peptide moiety can be measured by, for example, nuclear magnetic resonance (NMR).

<Other details of peptide-conjugated (meth)acrylic copolymer>

The number average molecular weight of the peptide-conjugated (meth)acrylic copolymer is preferably 5000 or more, more preferably 10000 or more, still more preferably 30000 or more, and particularly preferably 50000 or more, and is preferably 5000000 or less, more preferably 2500000 or less, and still more preferably 1000000 or less. When the number average molecular weight is the lower limit or more, the solubility of the peptide-conjugated (meth)acrylic copolymer in water can be further decreased, and the effects of the present invention can be more effectively exhibited. The relaxation time ($CS_2$), the absolute value (Z) of the difference, and the water absorption rate can be reduced. When the number average molecular weight is the upper limit or less, the solubility in an alcohol solvent can be further enhanced.

The number average molecular weight of the peptide-conjugated (meth)acrylic copolymer can be measured by, for example, the following method. The peptide-conjugated (meth)acrylic copolymer is dissolved in N,N-dimethylformamide (DMF) to prepare a 0.2 wt% solution of the peptide-conjugated (meth)acrylic copolymer. Next, evaluation is performed under the following measurement conditions using a gel permeation chromatography (GPC) measuring device (APC system, manufactured by Waters Corporation).

Column: HSPgel HR MB-M 6.0 × 150 mm
Flow rate: 0.5 mL/min
Column temperature: 40°C
Injection volume: 10 μL
Detector: RI, PDA
Standard sample: Polystyrene

A method for producing the peptide-conjugated (meth)acrylic copolymer is not particularly limited. The peptide-conjugated (meth)acrylic copolymer can be prepared, for example, as follows.

A monomer mixture containing a (meth)acrylate compound (A) and a (meth) acrylate compound (B) is polymerized to obtain a (meth)acrylate copolymer. The obtained (meth)acrylate copolymer is reacted with a peptide to obtain a peptide-conjugated (meth)acrylic copolymer.

(Other details of scaffold material for cell culture)

The scaffold material is used for culturing cells. The scaffold material is used as a scaffold for cells when the cells are cultured.

Examples of the cells include cells of animals such as humans, mouse, rat, pig, cow, and monkey. Examples of the cells include somatic cells and germ cells, and examples thereof include stem cells, progenitor cells, and mature cells. The somatic cells may be cancer cells.

Examples of the stem cells include somatic stem cells and embryonic stem cells, and examples thereof include neural stem cells, hematopoietic stem cells, mesenchymal stem cells (MSC), iPS cells, ES cells, Muse cells, embryonic cancer cells, embryonic germ stem cells, and mGS cells.

Examples of the mature cells include nerve cells, cardiomyocytes, retinal cells, renal cells, ovarian cells, and hepatocytes.

The scaffold material is preferably used for two-dimensional culture (plane culture), three-dimensional culture, or suspension culture of cells, more preferably used for two-dimensional culture (plane culture) or three-dimensional culture, and still more preferably used for two-dimensional culture.

The scaffold material is preferably used for serum-free medium culture. Since the scaffold material contains the peptide-conjugated (meth)acrylic copolymer, the adhesiveness of cells can be enhanced even in a serum-free medium culture containing no feeder cell or adhesive protein, and in particular, initial fixation rate after cell seeding can be enhanced. Since the scaffold material contains the peptide-conjugated (meth)acrylic copolymer, the effects of the present invention can be exhibited even in a serum-free medium culture.

The content of the peptide-conjugated (meth)acrylic copolymer in 100 wt% of the scaffold material is preferably 90 wt% or more, more preferably 95 wt% or more, still more preferably 97.5 wt% or more, particularly preferably 99 wt% or more, and most preferably 100 wt% (whole amount). When the content of the peptide-conjugated (meth)acrylic copolymer is the lower limit or more, the effects of the present invention can be more effectively exhibited. However, the content of the peptide-conjugated (meth)acrylic copolymer in 100 wt% of the scaffold material may be 100 wt% or less or 98 wt% or less.

The scaffold material may contain components other than the peptide-conjugated (meth)acrylic copolymer. Examples of the components other than the peptide-conjugated (meth)acrylic copolymer include polyolefin resins, polyether resins, polyvinyl alcohol resins, polyesters, epoxy resins, polyamide resins, polyimide resins, polyurethane resins, polycarbonate resins, polysaccharides, celluloses, polypeptides, and synthetic peptides.

It is preferable that the scaffold material does not substantially contain animal-derived raw materials. Since the scaffold material does not contain animal-derived raw materials, it is possible to provide a scaffold material having high safety and little variation in quality during production. The phrase "does not substantially contain animal-derived raw materials" means that the animal-derived raw materials in the scaffold material are 3 wt% or less. In the scaffold material, the animal-derived raw materials in the scaffold material are preferably 1 wt% or less and most preferably 0 wt%. That is, it is most preferable that the scaffold material does not contain any animal-derived raw materials.

The shape of the scaffold material is not particularly limited. The shape of the scaffold material may be a particulate shape, a fibrous shape, a porous shape, or a membrane shape.

The scaffold material is preferably a resin film. The resin film is preferably a resin film formed of the scaffold material. The resin film is a membrane-like scaffold material.

The thickness of the resin film is not particularly limited. The average thickness of the resin film may be 10 nm or more, 50 nm or more, or 500 nm or more, and may be 1000 um or less or 500 $\mu$m or less.

(Cell culture container)

A cell culture container preferably includes the above-described resin film in at least a part of a cell culture area. The cell culture container includes a container body and the above-described resin film, and the resin film is preferably disposed on a surface of the container body. The resin film is preferably a membrane-like scaffold material, and is preferably a scaffold material layer.

Fig. 1 is a cross-sectional view schematically illustrating a cell culture container according to an embodiment of the present invention.

A cell culture container 1 includes a container body 2 and a resin film 3. The resin film 3 is disposed on a surface 2a of the container body 2. The resin film 3 is disposed on a bottom surface of the container body 2. Cells can be cultured

in plane by adding a liquid medium is added to the cell culture container 1 and seeding cells such as cell mass on the surface of the resin film 3.

The container body may include a first container body and a second container body such as a cover glass on a bottom surface of the first container body. The first container body and the second container body may be separable. In this case, the resin film may be disposed on a surface of the second container body.

As the container body, a conventionally known container body (container) can be used. The shape and size of the container body are not particularly limited. As the container body, for example, a 2 to 384-well plate, a single-layer flask, a multi-layer flask, a multi-plane flask, a dish, a roller bottle, a bag, an insert cup, a microchannel chip, or the like can be used.

The material of the container body is not particularly limited, and examples thereof include resins, metals, and glass. Examples of the resin include polystyrene, polyethylene, polypropylene, polyethersulfone, polycarbonate, polyester, polyisoprene, a cycloolefin polymer, polyimide, polyamide, polyamideimide, a (meth)acrylic resin, an epoxy resin, and silicone.

(Cell culture microcarrier)

A cell culture microcarrier (hereinafter, may be abbreviated as "microcarrier") includes a base material particle and a coating layer covering an outer surface of the base material particle, and the coating layer is preferably formed of the scaffold material. The coating layer is preferably a scaffold material layer. As the base material particle, a conventionally known base material particle used in a microcarrier can be used. Examples of the base material particle include resin particles.

Fig. 2 is a cross-sectional view schematically illustrating a cell culture microcarrier according to an embodiment of the present invention.

A cell culture microcarrier 5 illustrated in Fig. 2 includes a base material particle 6 and a coating layer 7 covering an outer surface of the base material particle 6. The coating layer 7 is disposed on the surface of the base material particle 6 and is in contact with the surface of the base material particle 6. The coating layer 7 covers the entire outer surface of the base material particle 6.

(Method for culturing cells)

Cells can be cultured using the scaffold material, the resin film, and the microcarrier. The method for culturing cells is a method for culturing cells using the scaffold material. The method for culturing cells is preferably a method for culturing cells using the resin film, and is preferably a method for culturing cells using the microcarrier. Examples of the cells include the above-described cells.

The method for culturing cells preferably includes a step of seeding cells on the scaffold material. The method for culturing cells preferably includes a step of seeding cells on the resin film. The method for culturing cells preferably includes a step of seeding cells on the microcarrier. The cells may be a cell mass. The cell mass can be obtained by adding a cell detachment agent to a confluent culture container and uniformly crushing it by pipetting. The cell detachment agent is not particularly limited, but an ethylenediamine/phosphate buffer solution is preferable. The size of the cell mass is preferably 50 um to 200 μm.

Hereinafter, the present invention will be specifically described with reference to Examples. The present invention is not limited to the following Examples.

The content ratio of the structural unit derived from the (meth)acrylate compound and the content ratio of the peptide moiety in the obtained peptide-conjugated (meth)acrylic copolymer were measured by 1H-NMR (nuclear magnetic resonance spectrum) after dissolving the copolymer in DMSO-d6 (dimethyl sulfoxide).

(Example 1)

Preparation of peptide-conjugated (meth)acrylic copolymer:

Hexyl acrylate (48 parts by weight) and acrylic acid (52 parts by weight) were dissolved in 333 parts by weight of tetrahydrofuran to obtain an acrylic monomer solution. In the obtained acrylic monomer solution, 0.7 parts by weight of Irgacure 184 (manufactured by BASF) was dissolved, and the obtained solution was applied onto a PET film. A (meth)acrylic copolymer solution was obtained by irradiating the coated material with light having a wavelength of 365 nm at an integrated light quantity of 2000 mJ/$cm^2$ using a UV conveyor device ("ECS301G1" manufactured by Eye Graphics Co., Ltd.) at 25°C. The obtained (meth)acrylic copolymer solution was vacuum-dried at 80°C for 3 hours to obtain a (meth)acrylic copolymer.

As the peptide, a cyclic peptide having an amino acid sequence of Arg-Gly-Asp-Phe-Lys (five amino acid

residues, forming a cyclic skeleton by bonding Arg and Lys, Phe is a D form, described as c-RGDfK in the table) was prepared. As a condensing agent, 1-ethyl-3-(3-dimethylaminopropyl)carbodiimide hydrochloride was prepared. A (meth)acrylic copolymer (50 parts by weight) and a peptide (25 parts by weight) were dissolved in 1000 parts by weight of DMF (N,N-dimethylformamide) to prepare a first solution. A condensing agent (6 parts by weight) was mixed with 1000 parts by weight of DMF to prepare a second solution. The first solution and the second solution were mixed to prepare a solution containing the (meth)acrylic copolymer, the peptide, and the condensing agent. The obtained solution was reacted at 40°C for 2 hours, and a carboxyl group in the structural unit derived from acrylic acid of the (meth)acrylic copolymer and an amino group of Lys of the peptide were dehydrated and condensed, thereby obtaining a solution containing a peptide-conjugated (meth)acrylic copolymer.

Preparation of coating solution:

The obtained solution containing a peptide-conjugated (meth)acrylic copolymer was diluted 100 times with DMF, and added dropwise to a column packed with an ion exchange resin (manufactured by Organo Corporation) at a rate of 0.3 mL/min for washing. The solution after washing was vacuum-dried at 60°C for 3 hours to obtain a dried solid, and the dried solid was dissolved in ethanol to obtain a coating solution containing a peptide-conjugated (meth)acrylic copolymer and ethanol. The content of the peptide-conjugated (meth)acrylic copolymer in the coating solution was set to 0.1 wt%.

Preparation of cell culture container:

To each well of a 6-well plate, 20 μL of the obtained coating solution was applied by a cast coating method, and then ethanol was removed by vacuum drying at 60°C for 3 hours. In this way, a cell culture container in which a resin film (membrane-like scaffold material) formed of the scaffold material was disposed on the bottom surface of each well was obtained.

(Examples 2 to 9 and Comparative Examples 1 to 4)

Peptide-conjugated (meth)acrylic copolymers having the configurations shown in Tables 1 to 3 were obtained in the same manner as in Example 1, except that the kind or blending amount of the (meth)acrylate compound was changed. Cell culture containers were obtained in the same manner as in Example 1, except that the obtained peptide-conjugated (meth)acrylic copolymer was used.

(Evaluation)

(1) Number average molecular weight of peptide-conjugated (meth)acrylic copolymer

The number average molecular weight of the obtained peptide-conjugated (meth)acrylic copolymer was measured by the above-described method.

(2) Pulsed NMR

Using the obtained coating solution, measurement by a CPMG method using pulsed NMR was performed by the above-described method to determine the relaxation time ($CS_2$). Using the obtained coating solution, measurement by a Solid Echo method using pulsed NMR was performed by the above-described method to determine the relaxation time ($SwS_2$) and the relaxation time ($SdS_2$) . The absolute value (Z) of the difference between the relaxation time ($SwS_2$) and the relaxation time ($SdS_2$) was calculated.

Fig. 3 is a view showing a relationship between a relaxation time ($CS_2$) and an absolute value (Z) of a difference in scaffold materials for cell culture obtained in Examples and Comparative Examples.

(3) Water absorption rate

Using the obtained coating solution, the water absorption rate (wt%) of the scaffold material was calculated by the above-described method.

(4) Culture stability of cells (long-term culture stability)

The following liquid medium was prepared.

R-STEM (manufactured by ROHTO Pharmaceutical Co., Ltd.)

To the obtained cell culture container, 1 mL of phosphate buffered saline was added, the mixture was left to stand still in an incubator at 37°C for 1 hour, and then the phosphate buffered saline was removed from the cell culture container.

A cell suspension containing $5\times10^4$ cells (human fat-derived mesenchymal stem cell manufactured by LONZA KK., Model No.: PT-5006) was prepared in 1.5 mL of the liquid medium. This cell suspension was seeded into each well of a 6-well plate. Next, the 6-well plate was shaken 5 times to the right and left, and placed in an incubator at 37°C and a $CO_2$ concentration of 5% to perform culture.

The number of cells at 24 hours, 48 hours, and 72 hours after seeding of the cells was counted using Nucleo-Counter NC-3000 (manufactured by M&S TechnoSystems Inc.). When the doubling time of the cells between 24 hours and 48 hours after seeding was defined as T1, and the doubling time of the cells between 48 hours and 72 hours after seeding was defined as T2, T1 and T2 were calculated by the following formulas, respectively.

$$T1 = 24\ \log 2/(\log\ (N\ (48)/N\ (24))$$

$$T2 = 24\ \log 2/(\log\ (N\ (72)/N\ (48))$$

T1: Doubling time of cells from 24 hours to 48 hours after seeding
T2: Doubling time of cells from 48 hours to 72 hours after seeding
N (24): Cell number (cells) after 24 hours from seeding
N (48): Cell number (cells) after 48 hours from seeding
N (72): Cell number (cells) after 72 hours from seeding

From the doubling time (T1) and the doubling time (T2), a ratio (T1/T2) of T1 to T2 was calculated. The culture stability of cells was evaluated according to the following criteria. A larger ratio (T1/T2) means that the proliferation speed of cells is not decreased.

<Criteria for determining culture stability of cells (long-term culture stability)>

○○: The ratio (T1/T2) is 0.9 or more.
○: The ratio (T1/T2) is 0.7 or more and less than 0.9.
×: The ratio (T1/T2) is less than 0.7.

(5) Solubility in ethanol

The degree of solubility of the obtained scaffold material in ethanol was measured by the following method. The scaffold material was mixed with ethanol and stirred at 60°C, and after 30 minutes, the presence or absence of the undissolved scaffold material and the transparency of the solution were visually checked. The maximum solution concentration at which there was no undissolved scaffold material and the solution became clear was defined as the degree of solubility. The solubility in ethanol was evaluated according to the following criteria.

<Criteria for determining solubility in ethanol>

○○: The degree of solubility is 1 wt% or more.
○: The degree of solubility is 0.1 wt% or more and less than 1 wt%.
△: The degree of solubility is less than 0.1 wt%.

Compositions and results are shown in Tables 1 to 3 below.

[Table 1]

| | | | | Example 1 | Example 2 | Example 3 | Example 4 | Example 5 |
|---|---|---|---|---|---|---|---|---|
| (Meth) acrylic copolymer moiety | (Meth)acrylate compound (A) | Ethyl acrylate | mol% | | | | | |
| | | Butyl acrylate | mol% | | | | | 10 |
| | | Butyl methacrylate | mol% | | | | | |
| | | Hexyl acrylate | mol% | 30 | | | | |
| | | Dodecyl acrylate | mol% | | 30 | 45 | 65 | |
| | Other (meth) acrylate compound | Hydroxyethyl methacrylate | mol% | | | | | |
| | | Methoxytriethylene glycol acrylate | mol% | | | | | 25 |
| | (Meth) acrylate compound (B) | Acrylic acid | mol% | 65 | 65 | 50 | 30 | 60 |
| | | Methacrylic acid | mol% | | | | | |
| Peptide moiety | | c-RGDfK | mol% | 5 | 5 | 5 | 5 | 5 |
| Number average molecular weight of peptide-conjugated (meth)acrylic copolymer | | | | 50000 | 50000 | 50000 | 50000 | 50000 |
| Pulsed NMR | CPMG method | Relaxation time (CS2) | milliseconds | 0.90 | 1.65 | 1.51 | 0.74 | 4.30 |
| | Solid Echo method | Relaxation time (SwS2) | milliseconds | 0.052 | 0.059 | 0.059 | 0.042 | 0.532 |
| | | Relaxation time (SdS2) | milliseconds | 0.010 | 0.012 | 0.017 | 0.021 | 0.010 |
| | | Absolute value (Z) of difference between relaxation time (SwS2) and relaxation time (SdS2) | milliseconds | 0.042 | 0.047 | 0.042 | 0.021 | 0.522 |
| Water absorption ratio | | | wt% | 19.65 | 24.4 | 20.2 | 6.31 | 29.1 |
| Culture stability of cells (long-term culture stability) | | | | ○○ | ○○ | ○○ | ○○ | ○ |
| Solubility in ethanol | | | | ○ | ○○ | ○○ | ○ | ○○ |

[Table 2]

| | | | | Example 6 | Example 7 | Example 8 | Example 9 |
|---|---|---|---|---|---|---|---|
| (Meth) acrylic copolymer moiety | (Meth) acrylate compound (A) | Ethyl acrylate | mol% | | 30 | | |
| | | Butyl acrylate | mol% | 20 | | 40 | |
| | | Butyl methacrylate | mol% | | | | 20.5 |
| | | Hexyl acrylate | mol% | | | | |
| | | Dodecyl acrylate | mol% | | | | |
| | Other (meth) acrylate compound | Hydroxyethyl methacrylate | mol% | | | | 73 |
| | | Methoxytriethylene glycol acrylate | mol% | | | | |
| | (Meth) acrylate compound (B) | Acrylic acid | mol% | 75 | 65 | 55 | |
| | | Methacrylic acid | mol% | | | | 2.5 |
| Peptide moiety | | c-RGDfK | mol% | 5 | 5 | 5 | 4 |
| Number average molecular weight of peptide-conjugated (meth)acrylic copolymer | | | | 50000 | 50000 | 50000 | 50000 |
| Pulsed NMR | CPMG method | Relaxation time (CS2) | Milliseconds | 1.82 | 2.34 | 0.88 | 1.08 |
| | Solid Echo method | Relaxation time (SwS2) | Milliseconds | 0.062 | 0.440 | 0.047 | 0.012 |
| | | Relaxation time (SdS2) | Milliseconds | 0.010 | 0.010 | 0.011 | 0.010 |
| | | Absolute value (Z) of difference between relaxation time (SwS2) and relaxation time (SdS2) | milliseconds | 0.052 | 0.430 | 0.036 | 0.002 |
| Water absorption ratio | | | wt% | 22.2 | 23.4 | 15.4 | 1.1 |
| Culture stability of cells (long-term culture stability) | | | | ○○ | ○ | ○○ | ○○ |
| Solubility in ethanol | | | | ○○ | ○○ | ○ | △ |

[Table 3]

| | | | | Comparative Example 1 | Comparative Example 2 | Comparative Example 3 | Comparative Example 4 |
|---|---|---|---|---|---|---|---|
| (Meth) acrylic copolymer moiety | (Meth)acrylate compound (A) | Ethyl acrylate | mol% | | | 10 | |
| | | Butyl acrylate | mol% | | 10 | | |
| | | Butyl methacrylate | mol% | | | | |
| | | Hexyl acrylate | mol% | | | | |
| | | Dodecyl acrylate | mol% | | | | |
| | Other (meth) acrylate compound | Hydroxyethyl methacrylate | mol% | | | 85 | |
| | | Methoxytriethylene glycol acrylate | mol% | 70 | 85 | | 1.5 |
| | (Meth)acrylate compound (B) | Acrylic acid | mol% | 25 | | | |
| | | Methacrylic acid | mol% | | | | 93.5 |
| Peptide moiety | | c-RGDfK | mol% | 5 | 5 | 5 | 5 |
| Number average molecular weight of peptide-conjugated (meth) acrylic copolymer | | | | 50000 | 50000 | 50000 | 50000 |
| Pulsed NMR | CPMG method | Relaxation time (CS2) | Milliseconds | 5.97 | 12.69 | 7.81 | 14.23 |
| | Solid Echo method | Relaxation time (SwS2) | Milliseconds | 0.693 | 1.010 | 0.459 | 0.482 |
| | | Relaxation time (SdS2) | Milliseconds | 0.020 | 0.009 | 0.010 | 0.009 |
| | | Absolute value (Z) of difference between relaxation time (SwS2) and relaxation time (SdS2) | milliseconds | 0.672 | 1.001 | 0.449 | 0.473 |
| Water absorption ratio | | | wt% | 35.6 | 49.5 | 39.2 | 49.0 |
| Culture stability of cells (long-term culture stability) | | | | × | × | × | × |
| Solubility in ethanol | | | | ○○ | ○○ | ○○ | ○○ |

## EXPLANATION OF SYMBOLS

- 1: Cell culture container
- 2: Container body
- 2a: Surface
- 3: Resin film
- 5: Cell culture microcarrier
- 6: Base material particle
- 7: Coating layer

## Claims

1. A scaffold material for cell culture, comprising:

    a peptide-conjugated (meth)acrylic copolymer having a (meth)acrylic copolymer moiety and a peptide moiety bonded to the (meth)acrylic copolymer moiety,
    the following relaxation time ($CS_2$) being 5 milliseconds or less:
    the relaxation time ($CS_2$) being: a relaxation time of a CS component, which is a component having the lowest molecular mobility among three components, when the scaffold material for cell culture after being brought into contact with heavy water at 25°C for 18 hours is measured by a CPMG method using pulsed NMR, and the obtained free induction decay curve of spin-spin relaxation of $^1H$ is separated into three components.

2. The scaffold material for cell culture according to claim 1, wherein an absolute value of a difference between the following relaxation time ($SwS_2$) and the following relaxation time ($SdS_2$) is 0.6 milliseconds or less:

    the relaxation time ($SwS_2$) being: a relaxation time of an SwS component, which is a component having lower molecular mobility among two components, when the scaffold material for cell culture after being brought into contact with heavy water at 25°C for 18 hours is measured by a Solid Echo method using pulsed NMR, and the obtained free induction decay curve of spin-spin relaxation of $^1H$ is separated into two components, and
    the relaxation time ($SdS_2$) being: a relaxation time of an SdS component, which is a component having lower molecular mobility among two components, when the scaffold material for cell culture in a dry state is measured by a Solid Echo method using pulsed NMR, and the obtained free induction decay curve of spin-spin relaxation of $^1H$ is separated into two components.

3. The scaffold material for cell culture according to claim 2, wherein the absolute value of the difference between the relaxation time ($S_WS_2$) and the relaxation time ($SdS_2$) is 0.01 milliseconds or more and 0.3 milliseconds or less.

4. The scaffold material for cell culture according to any one of claims 1 to 3, wherein a water absorption rate is 35 wt% or less.

5. A scaffold material for cell culture, comprising:

    a peptide-conjugated (meth)acrylic copolymer having a (meth)acrylic copolymer moiety and a peptide moiety bonded to the (meth)acrylic copolymer moiety,
    a water absorption rate being 35 wt% or less.

6. The scaffold material for cell culture according to any one of claims 1 to 5, wherein the (meth)acrylic copolymer moiety has a structural unit derived from a (meth)acrylate compound (A) represented by the following Formula (A1) or the following Formula (A2):

[Chemical 1]

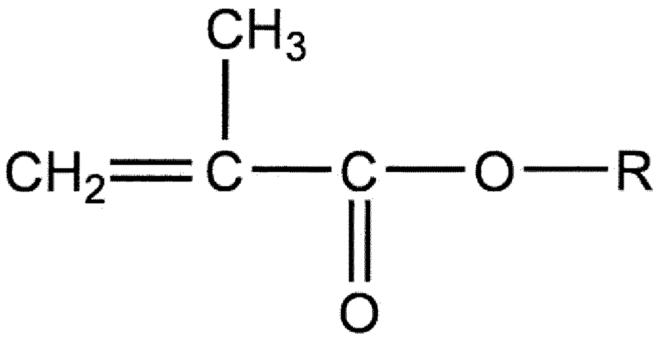

···(A1)

wherein R represents a hydrocarbon group having 2 or more and 18 or less carbon atoms,

[Chemical 2]

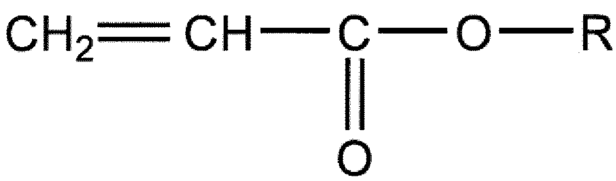

···(A2)

wherein R represents a hydrocarbon group having 2 or more and 18 or less carbon atoms.

7. The scaffold material for cell culture according to claim 6, wherein a content ratio of the structural unit derived from the (meth)acrylate compound (A) is 25 mol% or more and 98 mol% or less in 100 mol% of the total structural units of the (meth)acrylic copolymer moiety.

8. The scaffold material for cell culture according to any one of claims 1 to 7, wherein

the (meth)acrylic copolymer moiety has a structural unit derived from a (meth)acrylate compound (B) having a functional group capable of reacting with an amino group or a carboxyl group, and
in the peptide-conjugated (meth)acrylic copolymer, the peptide moiety is bonded to the functional group capable of reacting with an amino group or a carboxyl group.

9. The scaffold material for cell culture according to any one of claims 1 to 8, wherein a number average molecular weight of the peptide-conjugated (meth)acrylic copolymer is 5000 or more.

10. The scaffold material for cell culture according to any one of claims 1 to 9, wherein in the peptide-conjugated (meth)acrylic copolymer, a content ratio of the peptide moiety is 0.5 mol% or more and 25 mol% or less.

11. The scaffold material for cell culture according to any one of claims 1 to 10, wherein the peptide moiety has an RGD sequence.

[FIG. 1]

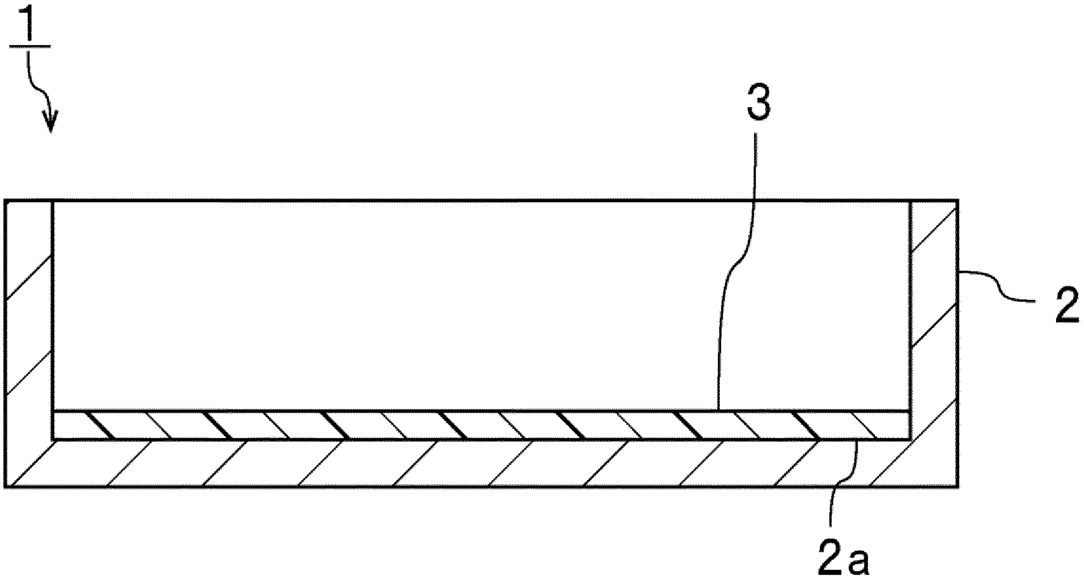


[FIG. 2]

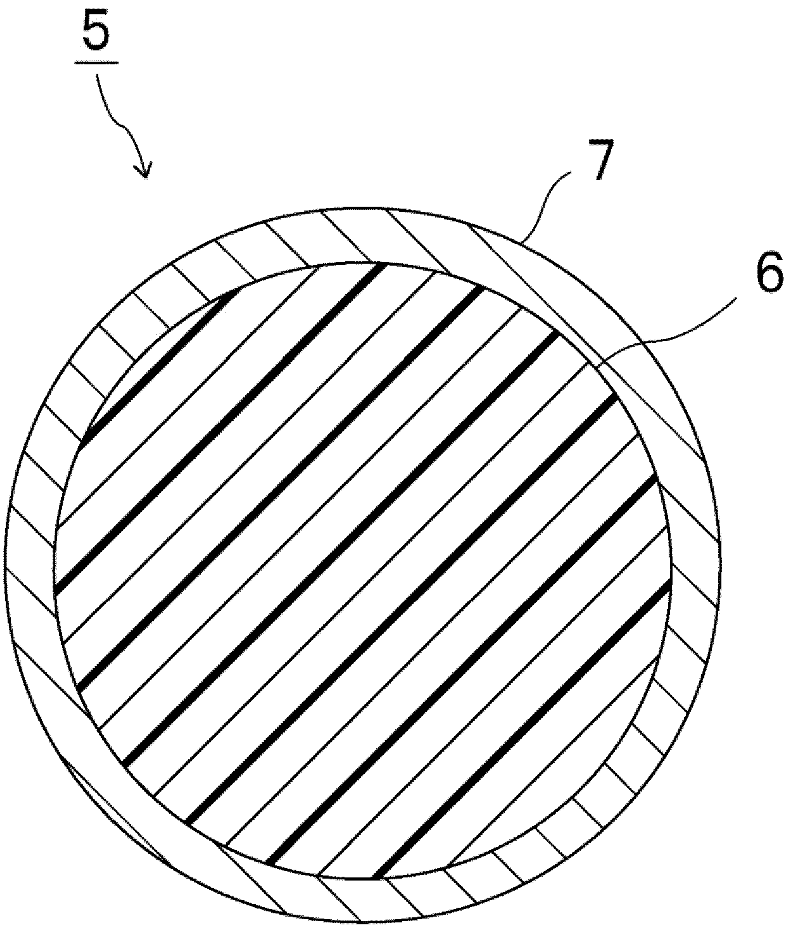

[FIG. 3]

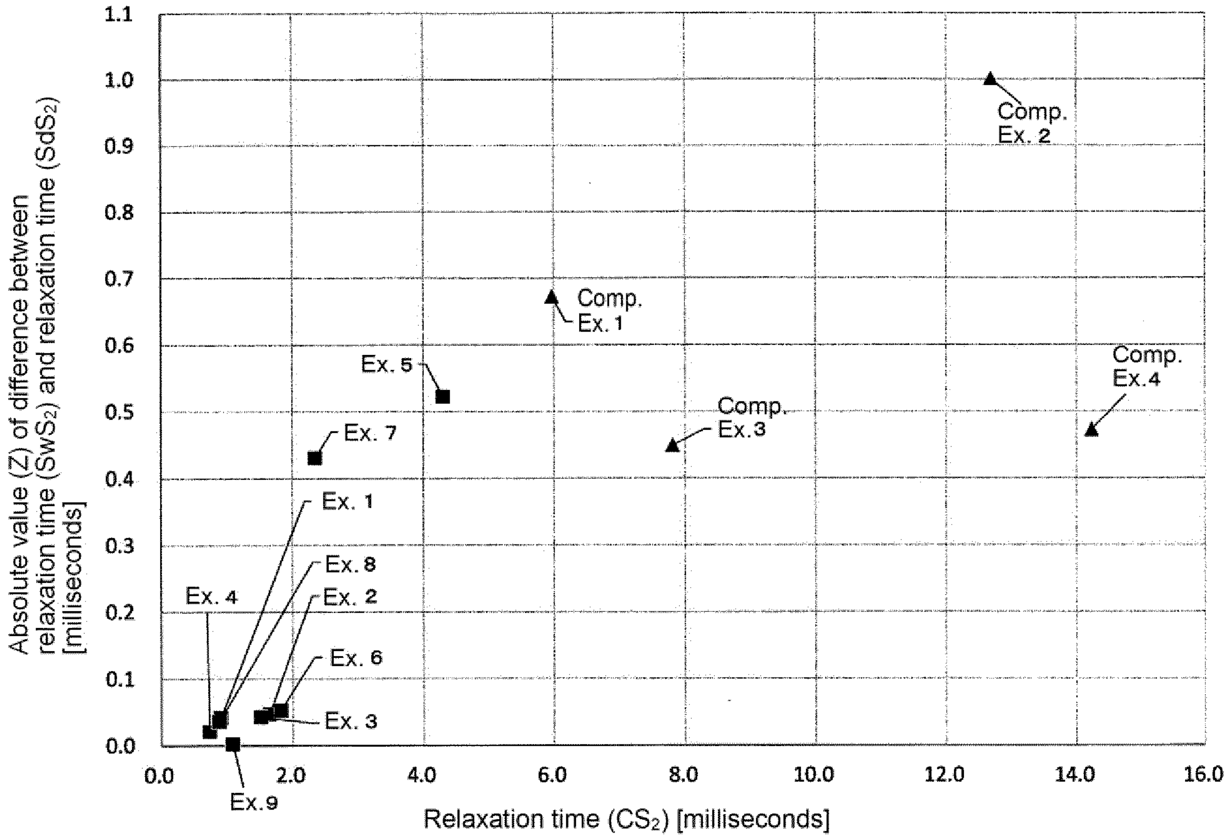

Absolute value (Z) of difference between relaxation time (SwS2) and relaxation time (SdS2) [milliseconds]
Relaxation time (CS2) [milliseconds]
1.1
1.0
0.9
0.8
0.7
0.6
0.5
0.4
0.3
0.2
0.1
0.0
0.0
2.0
4.0
6.0
8.0
10.0
12.0
14.0
16.0
Comp. Ex. 2
Comp. Ex. 1
Ex. 5
Comp. Ex.4
Comp. Ex.3
Ex. 7
Ex. 1
Ex. 8
Ex. 4
Ex. 2
Ex. 6
Ex. 3
Ex.9

## INTERNATIONAL SEARCH REPORT

International application No.
**PCT/JP2022/047838**

**A. CLASSIFICATION OF SUBJECT MATTER**

***C12M 3/00***(2006.01)i; ***C07K 5/09***(2006.01)i; ***C07K 5/10***(2006.01)i; ***C07K 5/11***(2006.01)i; ***C07K 7/06***(2006.01)i; ***C07K 17/08***(2006.01)i; ***C08F 261/12***(2006.01)i; ***C08G 81/02***(2006.01)i; ***C08L 101/06***(2006.01)i; ***C12M 1/00***(2006.01)i; ***C12N 1/00***(2006.01)i; ***C12N 5/071***(2010.01)i

FI: C12M3/00 A ZNA; C12N1/00 A; C07K5/10; C07K7/06; C12N5/071; C12M1/00 A ZNA; C08L101/06; C08F261/12; C07K17/08; C07K5/09; C07K5/11; C08G81/02; C12M1/00 C ZNA

According to International Patent Classification (IPC) or to both national classification and IPC

**B. FIELDS SEARCHED**

Minimum documentation searched (classification system followed by classification symbols)

C12M3/00; C07K5/09; C07K5/10; C07K5/11; C07K7/06; C07K17/08; C08F261/12; C08G81/02; C08L101/06; C12M1/00; C12N1/00; C12N5/071

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2020/230886 A1 (SEKISUI CHEMICAL CO., LTD.) 19 November 2020 (2020-11-19) claims, paragraphs [0027], [0052], [0072], [0077], [0079], [0083], [0101]-[0129], examples | 5-11<br>1-4 |
| X<br>A | JP 2021-23287 A (SEKISUI CHEMICAL CO., LTD.) 22 February 2021 (2021-02-22) claims, paragraphs [0027], [0047], [0052], [0080], [0082], [0089], [0092]-[0105], example 10 | 5-11<br>1-4 |
| A | JP 2016-190802 A (JSR CORP) 10 November 2016 (2016-11-10) entire text | 1-11 |
| A | JP 2021-3061 A (SEKISUI CHEMICAL CO., LTD.) 14 January 2021 (2021-01-14) entire text | 1-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 March 2023** | **14 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/047838**

**C. DOCUMENTS CONSIDERED TO BE RELEVANT**

| Category* | Citation of document, with indication, where appropriate, of the relevant passages | Relevant to claim No. |
|---|---|---|
| A | WO 2020/230884 A1 (SEKISUI CHEMICAL CO., LTD.) 19 November 2020 (2020-11-19) entire text | 1-11 |
| P, A | WO 2022/168870 A1 (SEKISUI CHEMICAL CO., LTD.) 11 August 2022 (2022-08-11) claims | 5-11 |

Form PCT/ISA/210 (second sheet) (January 2015)

**INTERNATIONAL SEARCH REPORT**

International application No.
**PCT/JP2022/047838**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   The “form of an Annex C/ST.25 text file” is replaced with the “form of ST.26.”

Form PCT/ISA/210 (continuation of first sheet) (January 2015)

**INTERNATIONAL SEARCH REPORT**
**Information on patent family members**

International application No.
**PCT/JP2022/047838**

| Patent document cited in search report | Publication date (day/month/year) | Patent family member(s) | Publication date (day/month/year) |
|---|---|---|---|
| WO 2020/230886 A1 | 19 November 2020 | US 2022/0227898 A1<br>claims, paragraphs [0029], [0056], [0074], [0079], [0081], [0085], [0103]-[0130], examples<br>EP 3971202 A1<br>KR 10-2022-0009367 A<br>CN 113166580 A | |
| JP 2021-23287 A | 22 February 2021 | (Family: none) | |
| JP 2016-190802 A | 10 November 2016 | (Family: none) | |
| JP 2021-3061 A | 14 January 2021 | (Family: none) | |
| WO 2020/230884 A1 | 19 November 2020 | US 2022/0348858 A1<br>Whole document<br>EP 3971201 A1<br>CN 113166201 A<br>KR 10-2022-0009366 A | |
| WO 2022/168870 A1 | 11 August 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012158235 A2
- JP H5292957 A


### Non-patent literature cited in the description


- *The Journal of Cell Biology,* September 1995, vol. 130 (5), 1189-1196
- *PROTEIN, NUCLEIC ACID, AND ENZYME,* 2000, vol. 45 (15), 2477
- *Pathophysiology,* 1990, vol. 9 (7), 527-535
- **OSAKA.** *Medical Center and Research Institute for Maternal and Child Health magazine,* 1992, vol. 8 (1), 58-66